# EUROPEAN PATENT APPLICATION

(11) **EP 1 229 117 A2**
(43) Date of publication of application: **07.08.2002**
(21) Application number: 01100080.9
(22) Date of filing: 10.01.2001
(51) Int. Cl.: C12N 15/12, C07K 14/705

(54) **Crystals of the human TRAIL/sDR5 (TNF-related apoptosis-inducing ligand/extracellular domain of DR5 receptor) complex, their three-dimensional structure and uses thereof**

(30) Priority: 29.12.2000 KR 2000085947
(71) Applicant: DONG WHA PHARMACEUTICAL INDUSTRIAL CO. LTD., Seoul 100-130 (KR); Pohang University of Science and Technology Foundation, Pohang, Kyongsangbuk-do 790-784 (KR)
(72) Inventor: Chung, Yong Ho, Anyang-si, Kyonggi-do,431-080 (KR); Ryu, Jei Man, Anyang-si, Kyonggi-do, 431-054 (KR); Hwang, Yun Ha, Kyonggi-do, 425-070 (KR); Yoon, Je In, Manan-ku, Anyang-si, 430-040 (KR); Oh, Byung-Ha, Pohang-si, Kyongsangbuk-do, 790-390 (KR); Cha, Sun-Shin, Eunpyong-ku, 122-081 (KR); Lim, Kyung Mook, Nowan-ku, Seoul 139-220 (KR)
(74) Representative: Grünecker, Kinkeldey, Stockmair & Schwanhäusser Anwaltssozietät

(57) **Abstract**

The present invention relates to a crystal of TRAIL (TNF related apoptosis including ligand)-sDR5 complex and TRAIL mutant protein. Particularly, the present invention relates to a method for preparing the crystal of human derived TRAIL protein and TRAIL receptor protein sDR5 complex and the crystal of TRAIL-sDR5 complex prepared by the method. The present invention also relates to a three-dimensional structure of TRAIL protein in the form of complex with TRAIL receptor sDR5 as well as TRAIL protein itself, and specific residues existed in the contact central region of AA" loop, which is involved in a specific polar interaction with sDR5 and plays an important role in the receptor binding affinity and the cytotoxic activity by analyzing the deletion mutant of TRAIL protein.

## Description

### FIELD OF THE INVENTION

The present invention relates to a crystal of TRAIL (TNF related apoptosis including ligand)-sDR5 complex and TRAIL mutant protein.

Particularly, the present invention relates to a method for preparing the crystal of human derived TRAIL protein and TRAIL receptor protein sDR5 complex and the crystal of TRAIL-sDR5 complex prepared by the method.

The present invention also relates to a three-dimensional structure of TRAIL protein in the form of complex with TRAIL receptor sDR5 as well as TRAIL protein itself, and specific residues existed in the contact central region of AA" loop, which is involved in a specific polar interaction with sDR5 and plays an important role in the receptor binding affinity and the cytotoxic activity by analyzing the deletion mutant of TRAIL protein.

### BACKGROUND

Apoptosis known to programmed cell death is different from cell death by necrosis and responsible for a proliferation and homeostasis of living things (Itoh, N. et al., *Cell*, 66:233-243, 1991). Many proteins are involved in the apoptotic process, and apoptotic signals are finally transferred to DNA in nuclei by binding an extracellular ligand to an intracellular protein domain. These receptors have a death domain in the intracellular protein domain, and directly act to apoptotic caspase machinery (Ashkenazi, A. et al., *Science*, 281:1305-1308, 1998).

Apoptosis plays an important role for removing activated lymphocytes, viral infected cells or transformed cells into tumor. Since apoptotic ligands interacting with apoptotic receptors induce the various form of cancer cells into apoptosis and independently act to p53 tumor suppressor factor which is used in chemical therapy or radiation therapy for cancer treatment, they are expected to its possibility of potential anticancer agents (Levine, A. J. et al., *Cell*, 88:323-331, 1997).

On the other hand, the TNF and TNF receptor (TNFR) superfamilies play important roles in regulating many biological functions, especially as prominent mediators of immune regulation, inflammatory responses, bone development and homeostasis (Cosman, D., *Stem Cells,* 12:440-455, 1994; Wong, B. R. et al., *Leukoc.* *Bio.*, 65:715-724, 1999). Ligands belonging to the TNF family are expected to function as a homotrimer as suggested by the crystal structures of a subset of the family (TNFα, TNFβ, CD40L and TRAIL). The monomeric unit of these proteins contains two β-pleated sheets that form a β-sandwich conforming to the jellyroll topology.

The TNFR family members are transmembrane proteins with several exceptions that contain an extracellular domain only. The extracellular domain of the receptors is characterized by the concatenated cysteine-rich domains (CRDs) (Bazan, J. F., *Curr. Biol.*, 3, 603-606, 1993) that are responsible for ligand binding. The intracellular domains of the receptor family members are not conserved, however, those of a subset of the receptors contain a common death domain. The major role of extracellular domain of the receptor is to provide a protein-protein interaction motif that recruits cellular partners and ultimately activates a protease cascade leading to apoptosis. Despite the same structural scaffolds, namely the β-sandwich for the TNF family and the concatenated CRDs for the TNFR family, the recognition between cognate ligands and receptors is achieved in a highly specific manner with typical dissociation constants in nanomolar range.

TRAIL is recently identified member of the TNF family (Wiley, S. R. et al., *Immunity*, 3(6):;673-682, 1995; Pitti, R. M. et al., *J. Biol. Chem.*, 271:12687-12690, 1996). It is a type II membrane protein that is proteolytically processed at the cell surface to form a soluble ligand (residues 114-281) (Mariani, S. M. et al., *Eur. J. Immunol.,* 28(57):1492-1498, 1998). An unique feature of TRAIL distinguished from those of other TNF family members is an insertion of 12-16 amino acids (depending on the proteins compared with) near the N-terminus (Cha, S. S. et al., *Immunity*, 11:253-261, 1999), called AA" loop, which is previously known to be important for the receptor binding of TNFs (Banner, D. W. et al., *Cell*, 73:431-445, 1993). TRAIL interacts with at least four different receptors that share a high sequence homology in the extracellular domains. Two of these are apoptosis-inducing receptors DR4 (Pan, G. et al., *Science*, 276:111-113, 1997) and DR5 (Pan, G. et al., *Science*, 277:815-818, 1997), both of which contain an intracellular death domain. The other two are nonsignaling decoy receptors, DcR1 and DcR2 (Marsters S. A. et al., *Curr. Biol.*, 7:1003-1006, 1997) that can bind to TRAIL but cannot trigger apoptosis.

The above decoy receptors are characterized that DcR1 lacks a cytoplasmic domain entirely, whereas DcR2 has a nonfunctional truncated death domain (Sheridan, J. P. et al., *Science*, 277:818-821, 1997). Another receptor, osteoprotegerin, binds to TRAIL with the dissociation constant of 400 nM, which is the lowest affinity of the known TRAIL receptors (Truneh, A. et al., *J. Biol. Chem.*, 275:23319-23325, 2000).

In contrast to TNFs and FasL, TRAIL has been known to induce apoptosis in a variety of tumor cells and some virally infected cells, but not in normal cells (Wiley, S. R. et al., *Immunity*, 3(6):673-682, 1995). Consistently, administration of TRAIL to mice bearing human tumors actively suppressed tumor progression (Walczak, H. et al., *Nat. Med.*, 5(2):157-163, 1999) and improved survival of the animal (Ashkenazi, A et al., *J. Clin. Invest.*, 104:155-162, 1999). Furthermore, repeated intravenous injections of TRAIL in nonhuman primates did not cause any detectable toxicity to normal tissues and organs including liver tissues (Ashkenazi, A. et al., *J. Clin. Invest.*, 104:155-162, 1999). However, susceptibility of human normal hepatocytes to TRAIL was reported very recently (Jo, M. et al., *Nat. Ned.*, 6:564-567, 2000). A working hypothesis for the selective antitumoral activity of TRAIL is that the decoy receptors are preferentially expressed in normal cells compared with tumor cells and interfere with the TRAIL action (Marsters, S. A. et al., *Curr. Biol.*, 7(12):1003-1006, 1997).

However, since TRAIL in the active form of homotrimer is converted into the inactive form during the purification, it has been reported that the use of TRAIL has a stability problem in clinical trials. To solve this problem, it has been tried that leucine zipper sequence is added to N-terminus of TRAIL to increase the stability (Walczak, H. et al., *Nat. Med.*, 5:157-163, 1999), and that TRAIL itself is administered to animal in dose of more 10 mg per kg in the clinical test (Ashkenazi, A. et al., *J. Clin. Invest.,* 104:155-162, 1999). The development of mutants like the above has been recognized as an important problem in the clinical application of TRAIL protein, and, on the basis of these results, it has been actively going on the study for developing the anticancer agents which cure the several kinds of cancers without any side effect.

Meanwhile, the study for investigating the molecular three-dimensional structure of biological polymers such as TRAIL has been in progress. The biological polymers exert their biochemical, biological functions through chemical or physical reaction by recognizing the smaller substance or other polymers. If the three-dimensional structure of recognition site for substances or other polymers is founded, it can be possible for inhibition, activation or conversion of biological polymer's function. When the three-dimensional structure of biological polymers are founded likethis, useful information for searching inhibitor proteins or developing mutant proteins having the desired function are easily obtained. In addition, this can establish the standard method for a new drug development based on the three-dimensional structure.

In a representative example for the development of the inhibitor proteins using the three-dimensional structure, there are AIDS curing agents based on the three-dimensional structure of HIV protease and influenza curing agents (clinical phase III) based on the three-dimensional structure of influenza viral sialidase. In addition, the molecular three-dimensional structure of hepatitis C viral helicase itself by investigating using X-ray beam determination method is open to the public as a patent, recently (WO 99/09148, 1999).

As a part of study for three-dimensional structure, the structure of TNFβ in complex with the extracellular domain of TNF-R55 (sTNFR55) had been determined (Banner, D. W. et al., *Cell*, 73:431-445, 1993). The structure of a TRAIL-receptor complex should provide valuable insights into molecular recognition between the two families that are expanding rapidly. Recently, two crystal structures of TRAIL in complex with the extracellular domain of DR5 (sDR5) were reported (Hymowitz, S. G. et al., *Mol. Cell*, 4:563-571, 1999; Mongkolsapaya, J. et al., *Nat . Struct. Biol.,* 6:1048-1053, 1999), and provided detailed views of the ligand-receptor interactions. However, neither of them sheds light on biological implication of the frame-insertion in the AA" loop.

In addition, to develop human derived TRAIL mutant having better stability, the present inventors has investigated the three-dimensional structure of TRAIL protein by crystallization and applied for a patent (KR Patent 99-35354). The above invention discloses that the human derived TRAIL gene is expressed in *E.coli,* the TRAIL protein is purified and crystallized, and the three-dimensional structure of TRAIL protein and TRAIL-sDR4 complex and come clear.

As a result of continuously studying the three-dimensional structure which regulates the binding specificity between TRAIL protein and TRAIL receptor pretein based on the above interaction, the present inventors has revealed that the specific residues in the AA" loop of TRAIL protein extensively interact with the receptors for TRAIL-sDR5 complex formation by preparing the crystal of TRAIL-sDR5 complex and analyzing the three-dimensional structure of crystal, and the specific residues of AA" loop plays an important role to the receptor binding affinity and the apoptotic activity.

### SUMMARY OF THE INVENTION

It is an object of this invention to provide a molecular strategy conferring the specificity between TNF and TNFR family.

It is a further object of this invention to provide a method of preparing the recombinant protein for cancer treatment, which has the better stability and the improved interaction with the receptors, by using the molecular strategy.

In accordance with the present invention, the foregoing objects and advantages are readily obtained.

The present invention provides a crystal of TRAIL-sDR5 complex.

This invention also provides the three-dimensional structure of TRAIL-sDR5 complex.

In addition, the present invention provides a binding mode of the TRAIL-sDR5 complex by comparing with TRAIL mutant protein.

Further features of the present invention will appear hereinafter.

### BRIEF DESCRIPTION OF THE DRAWINGS

FIG. 1a shows a three-dimensional structure of the TRAIL-sDR5 complex that three sDR5 molecules bind to three identical grooves existed between neighboring subunits of TRAIL.

FIG. 1b shows a stereo view of the superimposed TRAIL uncomplexed and complexed with sDR5 to compare the structural changes of TRAIL molecule induced by binding to sDR5 molecule.

FIG. 2a shows modular compositions of sDR5 in the three-dimensional structure of TRAIL-sDR5 complex of the present invention which has NI, CRD1 and CRD2 modules.

FIG. 2b shows the N1 module containing one disulfide bond in the three-dimensional structure of TRAIL-sDR5 complex of the present invention.

FIG. 3a shows a geometric structure of N1, CRD1 and CRD2 module of sDR5 in the three-dimensional structure of TRAIL-sDR5 complex of the present invention.

FIG. 3b shows a variation in the relative position of B2 module in CRD2 of sDR5 in the three-dimensional structure of TRAIL-sDR5 complex of the present invention.

FIG. 4a shows residues 131-135 of the AA" loop that penetrate into the central binding interface upon the three-dimensional structure of TRAIL-sDR5 complex of the present invention.

FIG. 4b shows an omit map of the residues 131-135 in the AA" loop upon the three-dimensional structure of TRAIL-sDR5 complex of the present invention.

FIG. 4c shows the interactions mediated by residues 131-135 in the AA" loop upon the three-dimensional structure of TRAIL-sDR5 complex of the present invention.

FIG. 5a shows a result of comparing the receptor binding affinity of TRAIL deletion mutant protein with that of wild-type TRAIL protein.

FIG. 5b shows a result of comparing the cytotoxic activity of TRAIL deletion mutant protein with that of wild-type TRAIL protein.

### DETAILED DESCRIPTION OF PREFERRED EMBODIMENTS

The present invention provides an expression vector containing TRAIL encoding gene, transformants incorporated with the expression vector, a preparing method of TRAIL protein using the transformants and the TRAIL protein produced by the preparing method.

The present invention also provides an expression vector containing sDR5 encoding gene, transformants incorporated with the expression vector, a preparing method of sDR5 protein using the transformants and the sDR5 protein produced by the preparing method.

In addition, the present invention provides a complex of TRAIL protein and sDR5 protein, a crystallizing method of the TRAIL-sDR5 complex, and a crystal of TRAIL-sDR5 complex prepared by the crystallizing method.

The present invention also provides a three-dimensional structure of TRAIL-sDR5 complex crystal.

Finally, the present invention provides a TRAIL deletion mutant and a preparing method thereof.

Hereinafter, the present invention is described in detail.

In one aspect, the present invention provides the expression vector containing the TRAIL encoding gene and the transformants incorporated with the expression vector.

Human derived TRAIL gene encodes polypeptide comprising 281 amino acids. As a result of comparing with the nucleic acid sequence of tumor necrosis factor, it has been found that a signaling site processed in cell exterior is amino acids 114-281 site represented by the SEQ. ID NO.1 (Pitti R. M. et al., *J. Biol. Chem.*, 271:12687-12690, 1996). Therefore, the present inventors construct the recombinant vector by using TRAIL gene fragment containing the amino acids 114-281.

The TRAIL gene fragment derived from mammals can be used, but preferably human derived TRAIL gene is used. The TRAIL gene fragment can be inserted into several kinds of *E.coli* expression vector. In on preferred embodiment of the present invention, the TRAIL gene fragment inserted into the recombinant vector is obtained from human derived placenta cDNA library by using PCR. The recombinant vector pET3a-TLS is prepared by inserting the TRAIL gene into T7 promoter downstream of *E.coli* expression vector pET3a. The transformant BL21-TLS(pET3a-TLS) is obtained by incorporating the recombinant vector pET3a-TLS into *E.coli* host BL21(DE3), and a mass-production of TRAIL protein is induced by culturing the transformant in the proper medium. For this, after the transformant cells are grown to an OD₆₀₀ of approximately 0.9, it is preferable to add IPTG as an inducer and culture at 22-37°C, and more preferable to culture at 25°C.

The *E.coli* transformant BL21-TLS(pET3a-TLS) has been deposited at Korean Culture Center of Microorganism on August 4, 1999 (Acession NO: KCCM-10169).

The preparing method of TRAIL-protein in the present invention is composed of the following steps;
1) during the cultivation of *E.coli* transformant BL21-TLS(pET3a-TLS), adding IPTG as the inducer to the medium and further culturing;
2) disrupting the cells by sonication and separating water-soluble fractions from cell lysate;
3) recovering soluble proteins from cell lysate by denaturing insoluble fractions and refolding; and
4) purifying the TRAIL protein described in the SEQ. ID NO.1 by performing cation exchange chromatography and sizing column chromatography with water-soluble fractions of cell lysate and the recovered water-soluble proteins.

After the transformant induced the expression of TRAIL protein in the step 1) is recovered and disrupted, the water-soluble fractions are used for the purification of TRAIL protein by using chromatography and the insoluble fractions are used for the purification of TRAIL protein from the dialyzed supernatant after protein denaturation, refolding and dialysis.

In the preparing method of TRAIL protein in the present invention, the chromatography for separation and purification can use the combination of cation exchange and sizing column chromatography. Particularly, the each fraction is eluted by using cation exchange chromatography with SP Sepharose solution. The presence or absence of TRAIL protein in the fractions is confirmed by SDS-PAGE, and the TRAIL containing fractions are recovered. After concentrating the active fraction, it can be purified by using sizing column such as 200 HR 10/30 sizing column similar to the above mentioned cation exchange chromatography. The purified TRAIL protein is confirmed by biological activating test.

The present invention also provides the expression vector containing sDR5 encoding gene, the transformants incorporated with the expression vector, a preparing method of sDR5 protein using the transformants and the sDR4 protein produced by the preparing method.

SDR5 gene represented by the SEQ. ID NO.2 is amplified by PCR from human fetal cDNA library. The recombinant vector pET-21b(+)-sDR5 is prepared by inserting the sDR5 gene into downstream of the T7 promoter on the expression plasmid pET-21b(+) and introduced in *E.coli* strain BL21(DE3). The *E.coli* transformant cells BL21(DE3)(pET21b+-sDR5) are grown to an OD₆₀₀ of approximately 0.4 in Luria-Bertani medium containing ampicillin, and the expression of sDR5 are induced by o.5 mM IPTG.

The sDR5 gene is confirmed by restriction enzyme map and nucleotide sequencing and the expressed protein is purified as follows. After the cultured cells are harvested and washed with 20 mM sodium phosphate buffer (pH 7.5), cells are resuspended in the same buffer and disrupted by sonication.

After centrifugation, supernatants are consecutively loaded on Ni-NTA column and the sDR5 protein is eluted with 20 mM sodium phosphate buffer (pH 7.5) containing imidazole. The eluted sDR5 protein is repeatedly loaded on Hi-Trap Q column and eluted again with 20 mM sodium phosphate buffer (pH 7.5) having 0-1.0 M linear gradient of NaCl.

The *E.coli* transformant BL21(DE3)(pET21b+-sDR5) has been deposited at Korean Culture Center of Microorganism on November 27, 2000 (KCCM-10230).

The preparing method of sDR5-protein in the present invention is composed of the following steps;
1) during the cultivation of *E.coli* transformant of the claim 2, adding IPTG as the inducer to the medium and further culturing;
2) disrupting the cells by sonication and recovering cell lysate; and
3) purifying the sDR5 protein having the amino acid sequence described in the SEQ. ID NO.2 by performing column chromatography with the cell lysate.

In addition, the present invention provides the complex of the TRAIL protein and sDR5 protein, the crystallizing method of the TRAIL-sDR5 complex and a crystal of TRAIL-sDR5 complex prepared by the crystallizing method.

The present inventors construct the TRAIL-sDR5 complex by mixing the TRAIL protein, which is expressed in the transformant containing TRAIL encoding gene and purified, with the sDR5 protein, which is expressed in the transformant containing sDR5 encoding gene and purified, in proper molar ratio.

It is preferable to mix the TRAIL protein with the sDR5 protein in 1:1 to 1:2 molar ratio. In a preferred embodiment, the TRAIL protein and the sDR5 protein are mixed in 1:1 molar ratio and incubated at 4°C for 12 hrs. After incubation, the mixture is loaded on column chromatography. The fractions containing the TRAIL-sDR5 complex are collected and used for crystallization.

Crystals of the TRAIL-sDR5 complex are obtained by hanging-drop vapor-diffusion method using 24 well Linbro plate at ambient temperature. According to the hanging-drop vapor-diffusion method, the crystals of complex are prepared by the following steps of;
1) adding 1 ml of precipitant solution containing 16% polyethyleneglycol 3000, 0.05 M sodium acetate (pH 4.5), 0.55 M sodium acetate and 0.6 M sodium chloride to the well;
2) mixing the precipitant solution with the protein solution in 1:1 molar ratio on the surface of cover slip; and
3) covering the well with the cover slip of the step 2) to be crystallized the protein.

The protein crystal produced by the above-mentioned method, belongs to the space group P21 with cell dimension a = 68.63, b = 124.81, c = 128.37 Å and β = 104.49°. The asymmetric unit contains two trimeric complexes of TRAIL and sDR5.

The crystal of TRAIL-sDR5 complex in the present invention shows totally different crystal packing from that of the other two crystals of the complex reported earlier. The crystal contains six molecules of TRAIL and sDR5 (two trimeric complexes) in the asymmetric unit and provides six independent views.

The structure of the TRAIL-sDR5 complex is determined by molecular replacement and refined to 2.2 Å resolution, and R factor of 21.2% and R_{free} of 29.1%. The final model of TRAIL-sDR5 complex in the present invention consists of TRAIL residues 120-135 and 146-281 in each monomer, sDR5 residues 21-128 of two copies and residues 21-102 and 116-123 of the other four copies, two metal ions, and 162 water molecules. Residues 1-20 of sDR5 are completely disordered.

The present invention provides the three-dimensional structure of TRAIL-sDR5 complex crystal.

The three-dimensional structures of TRAIL protein and sDR5 protein have been determined by the method comprising the following steps;
1) obtaining X-ray beam data from the protein crystal by using cooler or heavy metal inducer; and
2) determining the three-dimensional structure of protein by using protein modeling computer program after calculating electron density from X-ray data.

The heavy metal inducer of the step 1) contains K₂PtCl₆ and K₂HgI₄, and is used for calculating initial phase contrast information in order to collect X-ray beam data and construct the electron density map.

The computer program of the step 2) contains the well-known programs that determine the three-dimensional structure of protein based on the X-ray beam data, and uses the molecular replacement and multiple isomorphous replacement as principles. For example, CCP4 package, Program O, Program CHAIN, Program X-PLOR, Program DENZO or Program SCALEPACK can be used.

On the other hand, the three-dimensional structure of TRAIL-sDR5 complex is determined by the method comprising the following steps;
1) overlapping the three-dimensional structure of TRAIL protein with the three-dimensional structure of TNF-β/sDR5 complex in view of computer programs;
2) substituting the amino acid STNF-R55 in according to the amino acid order of TRAIL receptor DR5;
3) regulating backbone torsional angle of Glu 56 on the DR5 amino acid order in view of computer graphics to remove a collision of TRAIL and modeled DR5; and
4) performing energy minimization by computer program.

In the above step 1), the three-dimensional structure of TNF-β/sDR5 can use the previously know structure (Banner, D. W. et al., *Cell*, 73:431-445, 1993). The computer program of the step 1) and 3) can use QUANTA, that of the step 2) SWISS MODEL, that of the step 4) X-PLOR.

The three-dimensional structure of TRAIL and sDR5 protein or TRAIL-sDR5 complex may be stored and indicated on the computer device. In order to indicate the three-dimensional structure of the present invention on the computer device, the several kinds of software are induced.

As a result of determining the three-dimensional structure, the crystals of TRAIL-sDR5 complex belongs to the space group P2₁ with cell dimension a = 68.63, b = 124.81, c = 128.37 Å and β = 104.49°. The asymmetric unit contains two trimeric complexes of TRAIL and sDR5.

### 1) three-dimensional structure of TRAIL protein in TRAIL-sDR5 complex

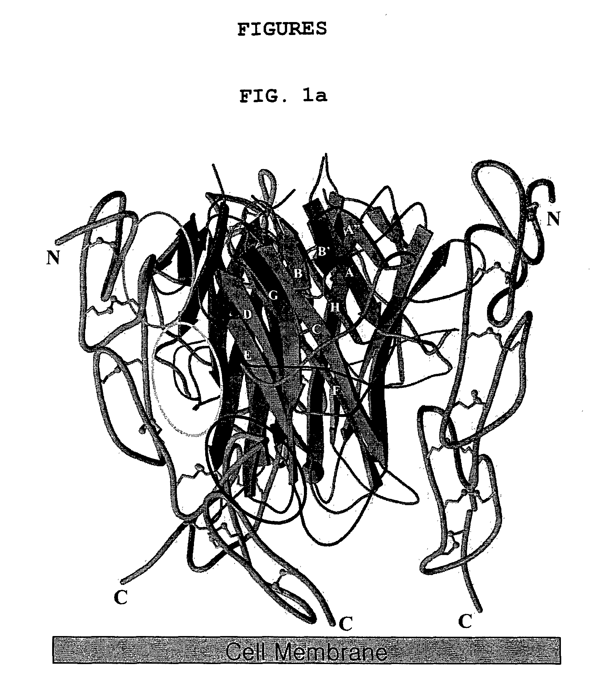

Structural variation between the uncomplexed and complexed TRAIL structures is concentrated in the loop regions, with the dramatic differences observed in the AA", CD and EF loops (see Fig. 1a). The CD and EF loops that are disordered in the uncomplexed structure become ordered on binding to sDR5. The AA" loop (residues 130-160) displays the most remarkable structural changes.

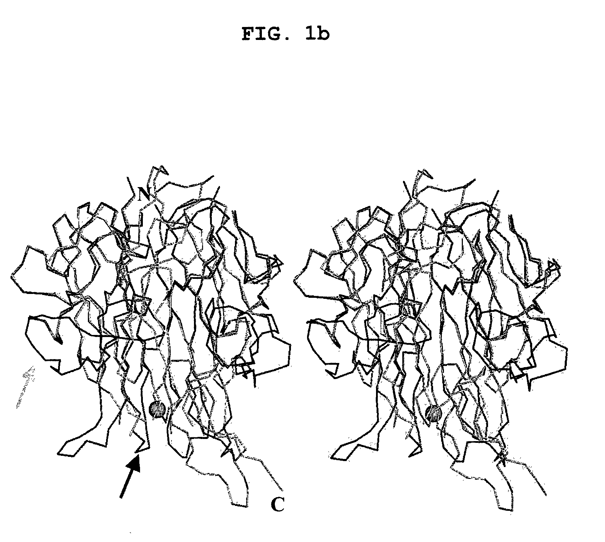

While the conformation of residues 146-160, which interact heavily with the core scaffold, is well maintained, the flexible segment composed of residues 130-145 (Cha, S. S. et al., Immunity, 11:253-261, 1999) undergoes a drastic positional change (see Fig. 1a). The initial part of the segment penetrates into the receptor-binding site and interacts with sDR5 (see Fig. 1b). This translocation is manifested by the strong electron densities for residues 130-135 that are observed in all six TRAIL molecules in the asymmetric unit.

### 2) three-dimensional structure of sDR5 protein in TRAIL-sDR5 complex

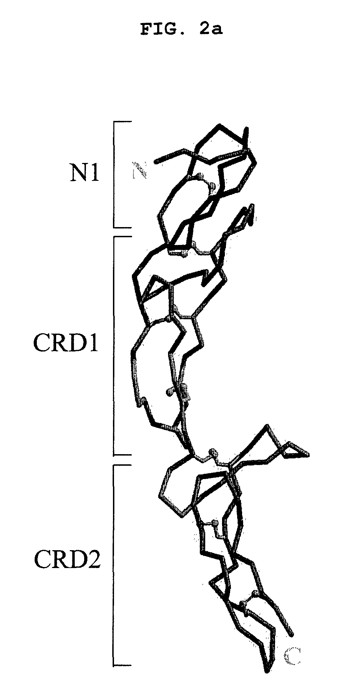

sDR5 has modular composition of N1 (residues 28-41), A1+B2 (CRD1; residues 44-84), and A1+B2 (CRD2; residues 86-125) (see Fig. 2a). A1 and B2 modules are composed of 12-17 and 21-24 amino acids, respectively. In the A1 modules, one disulfide bond exists between Cys1 and Cys2, while two disulfide bonds are formed between Cys1 : Cys3 and Cys2 : Cys4 in the B2 module.

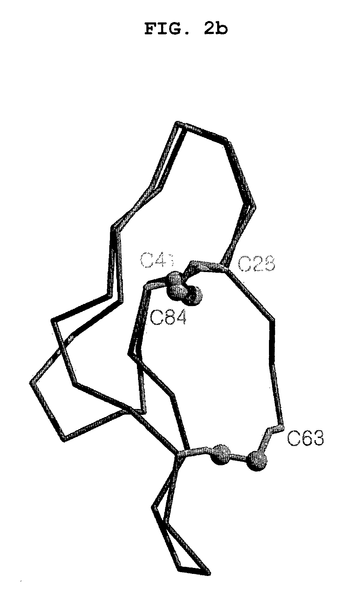

The N1 module has only one disulfide bond but is structurally related to the B2 module (see Fig. 2b). Residues 28-34 of the N1 module, which is structurally homologous to the B2 module, are involved in tight backbone interactions with the A1 module in CRD1 of sDR5, and these interactions are conserved in TNFR55.

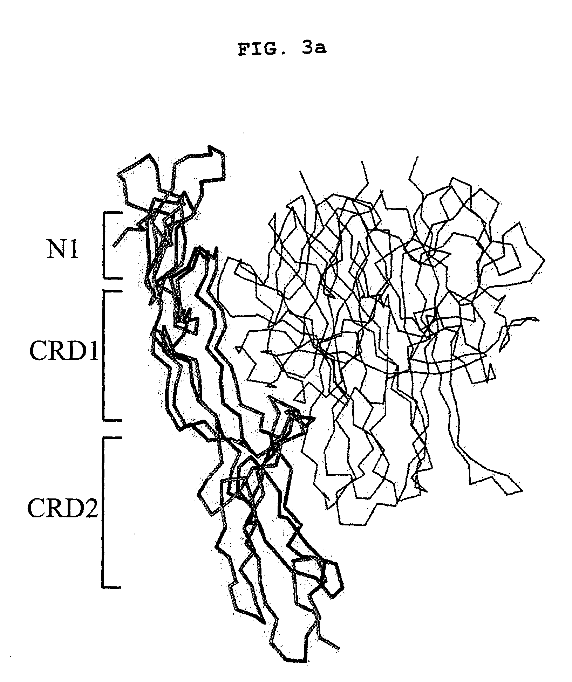

A superposition of the structure of TRAIL-sDR5 and TNFβ-sTNFR55 discloses that the N1 module and CRD1 in sDR5 and the corresponding B2 module and CRD in TNFR55 adopt virtually the same geometry on complex formation (see Fig. 3a).

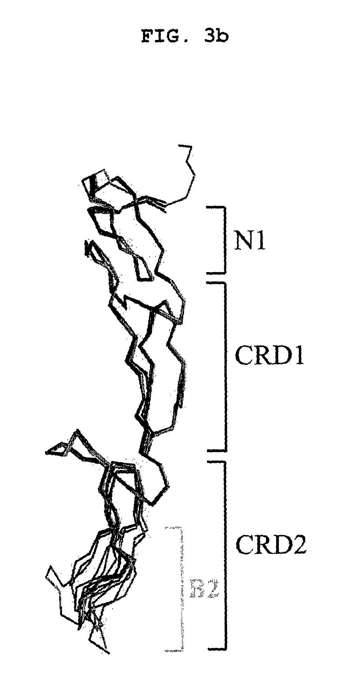

The six sDR5 modules in the asymmetric unit exhibit a large structural disparity in the B2 module of CRD2, while the B2 module and the successive three residues at the C-terminus in two sDR5 molecules are well ordered, those in the other four molecules are highly disordered (see Fig. 3b). The positions of the two ordered B2 modules in our structures are also different from those in the previously reported structures of the complex (see Fig. 3b).

### 3) Inetrface of the TRAIL-sDR5 complex

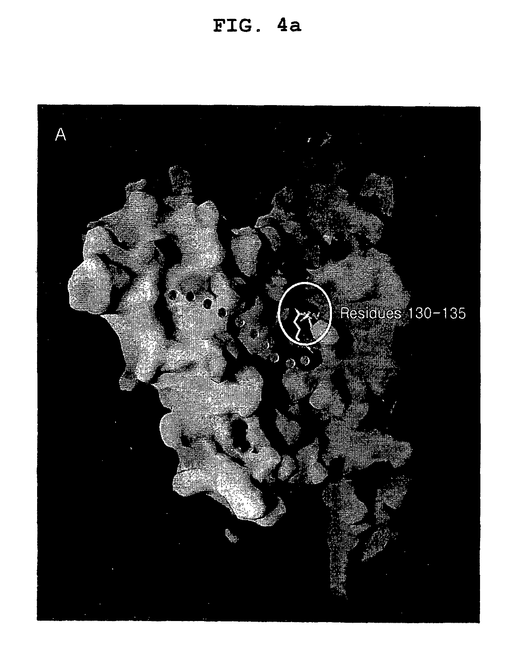

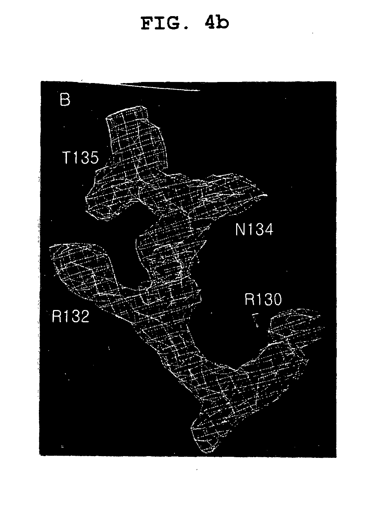

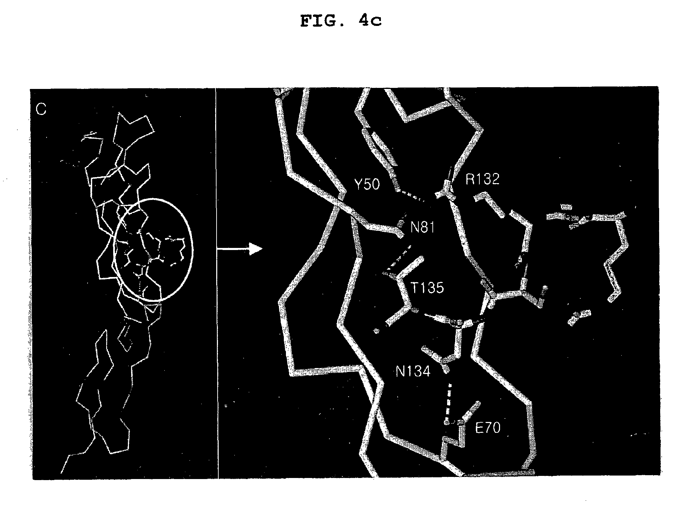

The central contact region, otherwise a central cavity like in the other three structures of the complexes, involves residues 131-135 of the AA" loop that penetrate into the central binding interface upon the complex formation (see Fig. 4a). The segment forms several specific polar interactions with sDR5, displaying strong electron densities even in an omit map (see Fig. 4b). The guanidino group of Arg 132 makes a polar interactions with Tyr 50 of sDR5. Asn 134 and Thr 135 interacts with Glu 70 and Asn 81 of sDR5, respectively (see Fig. 4c).

In addition, the present invention provides the deletion mutant of TRAIL protein and the preparing method thereof.

As a result of structural analyses of TRAIL-sDR5 complex, the present inventors has found that the residues 131-135 in the AA" loop of TRAIL forms several specific polar interactions with sDR5 receptor. The present inventors then construct a TRAIL mutant containing the deletion of residues 131-135 in the AA" loop to find the interaction of TRAIL protein and sDR5 receptor.

TRAIL deletion mutant (Δ132-135) is constructed by the overlapping PCR method (Du, Z. et al., BioTechniques, 18:376-378, 1995). The correct construction and expression are verified by nucleotide sequencing, mass spectrometry and circular dichroism spectroscopy.

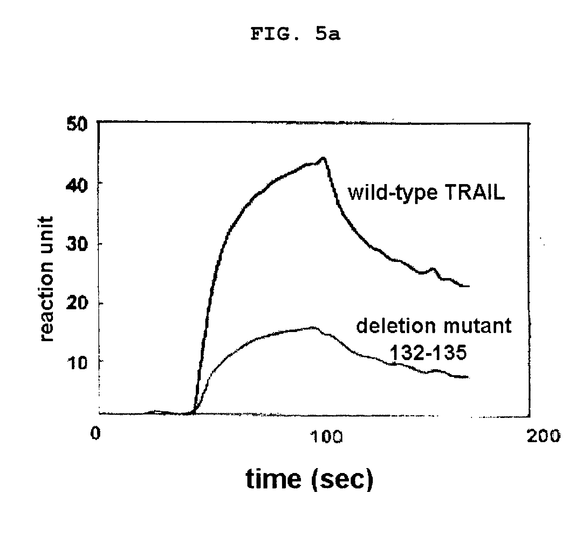

In order to compare the receptor binding affinity of TRAIL mutant with that of wild-type TRAIL, the present inventors measure the apparent K_{D} (dissociation constant). The apparent K_{D} of the TRAIL deletion mutant for sDR5, which is 2.94 (± 1.61) X 10⁻⁷ M, is 18-fold higher than that (1.59 [± 0.23] X 10⁻⁸ M) of the wild-type TRAIL (see Fig. 5a). This result means that the residues 131-135 in the AA" loop of TRAIL protein play an important role in the interaction with sDR5.

In order to examine the effect of the residues 131-135 of AA" loop to the cytotoxic activity, the present inventors measure the cytotoxic activities of TRAIL deletion mutant and wild-type TRAIL on human SK-HEP-1 hepatoma cells (ATCC HTB-52).

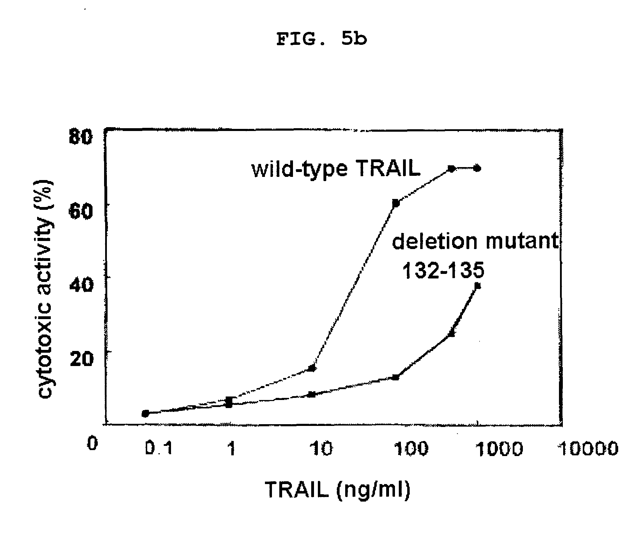

While the wild-type TRAIL reduces viability of cultured cells by 50% at about 50 ng/ml concentration, the deletion mutant resulted in less than 50% cell death even at 1000 ng/ml (see Fig. 5b). Therefore, the interactions at the central contact region in the residues 131-135 of AA" loop bear direct correlation with the activity of TRAIL.

These interactions also explain the activities of previously reported TRAIL variants containing a deletion on the AA" loop, one containing the deletion of residues 137-152 and the other containing Ser-Leu-Leu sequence instead of 135-153 to mimic the short AA" loop of TNFβ. Both mutants exhibited a drastically reduced apoptotic activity (Cha, S. S. et al., *Immunity*, 11:253-261, 1999) or affinity for sDR5 (Mongkolsapaya, J. et al., *Nature Struct. Bio.*, 6:1048-1053, 1999). The weak interaction of Arg 149 alone cannot explain the defective activities.

These, however, can be explained by the expected loss of the movable nature of the AA" loop when it is curtailed. Although the shortened AA" loops of the deletion mutants contain one fewer or all the residues that interact with the receptor, they could not be translocated into the central binding region, especially because the second half of the AA" loop is held tight on the outer β-sheet of TRAIL.

Together with the previous and the new deletion analyses, the three-dimensional structure of TRAIL-sDR5 complex in the present invention underscores the critical role of the frame insertion in the receptor recognition played by providing the conformational flexibility of the AA" loop. Namely, it is determined that the frame insertion is involved in the interaction between TRAIL and TRAIL receptor and plays an important role in the recognition of two molecules.

The present invention also provides the developing method of recombinant proteins that have the better stability or the improved cytotoxic activity of TRAIL protein.

The recombinant proteins that are more stable or active than the wild-type protein can be developed by using the three-dimensional structure obtained from the structural analysis of the present invention and the well-known mutant protein.

Particularly, the homotrimer structure of TRAIL is essential to the TRAIL activity, and the force forming this structure is due to the various interactions between the amino acids existed in the interface of homotrimer or homodimer. It is well known that the presence and its size of cavity in the protein are closely related to the stability of protein, and the stability is increased by filling the cavity (Vlassi et al., *J. Mol. Biol.*, 285:817-827, 1999; Takano, K. et al., *Mol. Biol.*, 254:62-76, 1995; Erikson, A. E. et al., *Science*, 255:178-183, 1992).

According to the present invention, it is confirmed that the specific cavity exists in the interface of TRAIL homotrimer, and a distance between the amino acids forming the around of the largest cavity is 5 Å. Therefore, it can be possible to perform the mutation of homotrimer interface area by using the specific cavity of homotrimer interface which is determined in the three-dimensional structure of TRAIL.

In addition, it is previously reported that the mutant tumor suppressor having the changed receptor binding affinity is produced by substituting the amino acids existed in the receptor binding site of tumor suppressor (Cha, S. S. et al., *J. Biol. Chem.*, 273:2153-2160, 1998). So, the mutants changed its binding affinity between TRAIL and its receptor are easily constructed by using the major amino acids of receptor binding site formed in the three-dimensional structure of TRAIL-sDR5 complex in the present invention, particularly, the specific residues in the AA" loop.

In order to increase the stability of TRAIL protein, when the formation of homotrimer is increased by modifying the specific residues of AA" loop, the homotrimer interface or the homodimer interface, it can be modified by
1) changing the amino acid of AA" loop to increase the various interaction between amino acids or form the binding site of metal ion or the disulfide bonding; and
2) changing the corresponding amino acid of homotrimer interface or homodimer interface to increase the various interactions between amino acids or form the binding site of metal ion or disulfide bond, or fill the cavity.

In addition, when the receptor binding sites of TRAIL is manufactured to increase the cavity of TRAIL protein, it can be possible to modify the amino acid residues corresponding to the AA" loop in order to increase the various site of metal ion or disulfide bond. The mutation of the amino acid residues in the present invention is performed by substitution, insertion or deletion of one or more amino acid.

### EXAMPLES

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Expression and purification of TRAIL protein

### <1-1> Construction of transformant expressing TRAIL protein

To construct transformants expressing TRAIL protein, DNA fragment described in the SEQ. ID NO.1 which encodes the amino acid residues from 114 to 281 was amplified from λgtII human derived placenta cDNA library (Clontech) by PCR. The recombinant vector pET3a-TLS was constructed by inserting the amplified DNA fragment into T7 promoter downstream of expression vector pET-3a (Novagen). *E.coli* BL21(DE3) transformant was prepared by introducing the recombinant vector and selected by culturing in LB medium containing 0.5 mg/ml of ampicillin at 37°C. It was confirmed that the selected transformant was introduced with the recombinant vector by analyzing the restriction enzyme map and sequencing amino acid.

The *E.coli* transformant was named as BL21-TLS(pET3a-TLS) and deposited at Korean Culture Center of Microorganism on August 4, 1999 (Accession NO: KCCM-10169).

### <1-2> Expression of TRAIL protein from transformant

To express the TRAIL protein from TRAIL transformant prepared in the Example <1-1> in a large quantity, TRAIL gene expression was induced by culturing the TRAIL transformant in LB medium containing 0.1 mg/ml of ampicillin and 0.5 mM IPTG (isopropyl-β-D-thiogalactoside) at 37°C. After cultivation at 37°C for 4 hrs, cells were collected by centrifugation, washed with 20 mM sodium phosphate buffer (pH 7.5), suspended in the same buffer, and disrupted by sonication.

### <1-3> Purification of TRAIL protein

Cell lysate of TRAIL transformant obtained from the Example <1-2> was separated into water-soluble fraction and water-insoluble fraction, and the water-soluble fraction was used for protein purification. TRAIL inclusion body was collected from the water insoluble fraction by centrifugation, and denatured by using buffer 1 comprising 20 mM sodium phosphate buffer (pH 7.6), 6 M guanidine hydrochloride (HCl) and 1 mM dithiothreitol.

The denatured protein was refolded by using 10-fold diluted solution of buffer 2 containing 20 mM sodium phosphate buffer (pH 7.6), 1 mM dithiothreitol and dialysed by using the same diluted solution at 4°C overnight. After the precipitate formed for dialysis was removed, the supernatant was used for the purification of TRAIL protein by using the chromatography. Particularly, the fraction loaded on SP Sepharose high-speed column (Pharmacia Biotech), and washed with 20 mM sodium phosphate buffer (pH 7.5). TRAIL protein was eluted with the buffer 2 containing sodium chloride, which concentration was gradually increased by using 0.1-1.0 M of sodium chloride gradient solution.

After confirming the elution of TRAIL protein by SDS-PAGE with the eluted fractions, the fraction containing TRAIL protein was concentrated to the concentratation of 10 mg/ml, and the concentrated solution was loaded on sizing column 200 HR (Pharmacia). The TRAIL protein was eluted with the buffer 2, and the elution was confirmed by SDS-PAGE. The TRAIL containing fraction was dialyzed with the buffer containing 10 mM HEPES (Ph 7.6), 1 mM dithiothreitol and 0.3 M sodium chloride, concentrated to the concentration of 12 mg/ml, and used for crystallization.

### Example 2: Expression and purification of sDR5 protein

### <2-1> Construction of sDR5 transformant

DNA fragment described in the SEQ. ID NO.2 which encodes the sDR5 amino acid residues from 1 to 130, was amplified from human derived fetal cDNA library by PCR. A recombinant vector was constructed by inserting the amplified DNA fragment into the T7 promoter downstream of expression vector pET-21b(+), introduced in *E.coli* BL21(DE3). The *E.coli* transformant was confirmed its transformation of recombinant vector by analyzing restriction enzyme map and nucleic acid sequencing. The E.coli transformant was named as BL21(DE3) (pET21b+-sDR5) and deposited at Korean Culture Center of Microorganism on November 27, 2000 (Accession NO: KCCM-10230).

### <2-2> Expression of sDR5 protein from transformant

In order to express the sDR5 protein from the sDR5 transformant prepared in the Example <2-1>, cells were grown to an OD₆₀₀ of approximately 0.4 in Luria-Bertani medium containing 0.1 mg/ml ampicillin at 37 °C, and the expression of sDR5 was induced by 0.5 mM IPTG. After 7 hrs induction at 27°C, cells were harvested and resuspended in a 20 mM sodium phosphate buffer (pH 7.5) and disrupted by sonication.

### <2-3> Purification of sDR5 protein

The supernatant was separated from the cell lysate of sDR5 transformant obtained from the Example <2-2> by centrifugation, and loaded on Ni-NTA column. SDR5 protein was eluted with 20 mM sodium phosphate buffer (pH 7.5) containing 100 mM imidazole. When the eluted sDR5 protein was loaded on Hi-Trap Q column, the sDR5 protein absorbed to the column, and was eluted with linear gradient concentration of 0 to 1.0 M by dissolving NaCl in 20 mM sodium phosphate buffer (pH 7.5).

### Example 3: Construction and purification of TRAIL-sDR5 complex

The TRAIL and sDR5 protein purified from the Example 1 and 2 were mixed in 1:1 molar ratio. After 12 hrs incubation at 4°C, the mixture was loaded on Superdex 200 HR sizing column (Pharmacia). The TRAIL-sDR5 complex was eluted by washing the column with buffer 2, and the presence of complex was confirmed by SDS-PAGE with eluted fractions. The feactions containing the TRAIL-sDR5 complex were collected, dialyzed with buffer containing 10 mM HEPES (pH 7.6), 1 mM dithiothreitol and 0.3 M sodium chloride, and used for crystallization to the concentration of 10 mg/ml.

### Example 4: Crystallization of TRAIL-sDR5 complex

Crystals of the TRAIL-sDR5 complex was obtained by hanging-drop vapor-diffusion method using 24 well Linbro plate at ambient temperature. According to the hanging-drop vapor-diffusion method, the crystals of complex was prepared by the following steps of;
1) adding 1 ml of precipitant solution containing 16% polyethyleneglycol 3000, 0.05 M sodium acetate (pH 4.5), 0.55 M sodium acetate and 0.6 M sodium chloride to the well;
2) mixing the precipitant solution with the protein solution in 1:1 molar ratio on the surface of cover slip; and
3) covering the well with the cover slip of the step 2) to be crystallized the protein.

The crystals prepared by the above method belonged to the space group P2₁ with cell dimensions a = 68.63, b = 124.81, c = 128.37 Å and β = 104.49°. The asymmetric unit contains two trimeric complexes of TRAIL and sDR5.

The crystals of the TRAIL-sDR5 complex of the present invention had a totally different crystal packing from those of the other two crystal of the complex reported earlier (Hymowitz, S. G. et al., *Cell*, 4:563-571, 1999; Mongkolsapaya, J. et al., *Nature Struct. Bio.*, 6:1048-1053, 1999). The crystals of the contained six molecules of TRAIL and sDR5 (two trimeric complexes) in the asymmetric unit and provide six independent views.

The structure of the TRAIL-sDR5 complex is determined by molecular replacement (Rossmann, M. G. et al., (ED) The molecular replacement method., Gordon and Breach, New York, 1972) and refined to 2.2 Å resolution, and R factor of 21.2% and R_{free} of 29.1%. The final model of TRAIL-sDR5 complex in the present invention consists of TRAIL residues 120-135 and 146-281 in each monomer, sDR5 residues 21-128 of two copies and residues 21-102 and 116-123 of the other four copies, two metal ions, and 162 water molecules. Residues 1-20 of sDR5 are completely disordered.

### Example 5: Structure determination and refinement of the crystals of the TRAIL-sDR5 complex

For collecting X-ray beam data of the TRAIL-sDR5 complex, the crystal was frozen at 100° K of nitrogen gas after being briefly immersed in a cryoprotectant solution containing 15% glycerol, 16% polyethylene glycol 3000, 0.05 M sodium acetate (pH 4.5), 0.3 M sodium acetate and 0.3 M NaCl. A 2.2 Å data set was collected using X-ray beam (λ = 1.0 Å) from the BL6A beamline at the Photon Factory (Japan) and processed with the programs DENZO and SCALEPACK (Otwinowski, Z. et al., *Methods Enzymol.*, 276:307-326, 1997).

Crystallographic refinements, iterative map calculations and model building were done using the programs X-PLOR (Brunger, A. T., Yale University Press, New Haven, CT, 1992) and Program O (Jones, T. A. et al., O version 5.9, The manual, Uppsala University, Uppsala, Sweden). The 2-fold NCS restrains were maintained only for TRAIL until the last refinement cycle. From the beginning of the refinement, 5% of the total reflections were set aside for monitoring R_{free} value. The final crystallographic statistics are shown in Table 1.

**<Table 1>**

| Crystal structure determination and refinement statistics | |
|---|---|
| Data Collection and Refinement (F>1σ) | |
| Resolution (Å) | 2.20 |
| R_{sym} (%) | 6.1 |
| Completeness (F>1σ) | 93.1 |
| R Factor | 21.2 |
| R_{free} | 29.1 |
| Number of protein atoms | 12,837 |
| Water molecules | 162 |
| Metal ion | 2 |
| Average B-factor (Å²) | 21.81 |
| Rmsd bond lengths (Å) | 0.011 |
| Rmsd bond angles (degree) | 1.772 |

| Ramachandran Plot (%) | |
|---|---|
| Most favored region | 77 |
| Additionally allowed region | 23 |
| Disallowed region | 0 |
| R_{sym} = Σ[I_{obs}-I_{avg}]/ΣI_{obs}, and R factor = Σ[Fₒ-F_{c}]/ΣFₒ. The R_{free} was calculated with 5% of the data. | |

Using the uncomplexed TRAIL structure (Cha, S. S. et al., *Immunity*, 11:253-261, 1999) as a search model, two promising solutions were found using the CCP4 version of AMoRe. The favorable crystal packing of the two solutions with marginal space for sDR5 molecules confirmed the correctness of the solutions. The initial electron densities obtained by solvent flattening and 2-fold noncrystallographic symmetry (NCS) density averaging using the CCP4 suite (CCP4, *Acta. Crystallogr. D.*, 50:760-763, 1994) allowed us to build a partial sDR5 model. As the manual fitting and structure refinement were in progress, weak but continuous electron densities extended from Arg 130 became stronger, obviously showing features of the side chains of residues 130-135 on the AA" loop.

### <5-1> Structure of sDR5

The Fig. 1 shows a three-dimensional structure of TRAIL-sDR5 complex obtained from the Example 3.

The overall structure of TRAIL in a complex with sDR5 was virtually the same as that of the uncomplexed TRAIL structure (Cha, S. S. et al., *Immunity,* 11:253-261, 1999). A TRAIL monomer contains two antiparallel (β-pleated sheets that form a β sandwich as a core scaffold, and interacted with the adjacent subunits in a head-to-tail fashion to form a bell-shaped homotrimer. Structural variation between the uncomplexed and complexed TRAIL structures is concentrated in the loop regions, with the dramatic differences observed in the AA", CD and EF loops (Fig. 1a). The CD and EF loops that are disordered in the uncomplexed structure become ordered on binding to sDR5. The AA" loop (residues 130-160) displays the most remarkable structural changes.

While the conformation of residues 146-160, which interact heavily with the core scaffold, is well maintained, the flexible segment composed of residues 130-145 (Cha, S. S. et al., *Immunity*, 11:253-261, 1999) undergoes a drastic positional change (Fig. 1a). The initial part of the segment penetrates into the receptor-binding site and interacts with sDR5 (Fig. 1b). This translocation is manifested by the strong electron densities for residues 130-135 that are observed in all six TRAIL molecules in the asymmetric unit.

TRAIL monomer contained single cysteine, Cys 230. The cysteine residues from three monomers are close to each other perpendicular to the molecular three-fold axis, forming a triangular geometry with three sulfur as apexes. A strong electron density observed near the cysteinyl sulfur at the first cycle of the structure refinements indicated a metal binding. This is different from the structure of uncomplexed TRAIL (Cha, S. S. et al., *Immunity*, 11:253-261, 1999) and that of TRAIL-sDR5 complex (PDB code: 1D4V) of Mongkolsapaya et al. (1999), both of which are determined with refolded TRAIL. It is, however, consistent with the structure of TRAIL-sDR5 complex (PDB code: 1D0G) of Hymowitz et al. (1999) in which the three cysteines chelate Zn²⁺ with the fourth coordination arm, Cl⁻.

The present invention demonstrates that the frame insertion specifically existed in TRAIL molecule plays an important role in the interaction with the receptor in the three-dimensional structure of the TRAIL-sDR5 complex, however, the previous report about the three-dimensional structure of TRAIL-sDR5 complex dose not. Therefore, the present invention suggest that the three-dimensional structure of the TRAIL-sDR5 complex can be used for production of TRAIL mutant by changing the amino acid in the inserted frame in coincident with the medicinal purpose.

Removal of the Zn²⁺ by chelating agents is shown to result in 90-fold decrease in apoptotic activity and a marked decrease in melting temperature (Hymowitz, S. G. et al., *Biochemistry*, 39:633-640, 2000). Previously, the present inventors have reported that refolded Zn²⁺- unbounded TRAIL slowly converts into inactive monomeric or dimeric form (Cha, S. S. et al., *Immunity*, 11:253-261, 1999). Although the zinc binding site is buried in the trimeric interface (Fig. 1a), and thus Zn²⁺- binding cannot directly influence the binding affinity of TRAIL of the receptors, it must be critical for maintaining the active trimeric structure of the protein (Hymowitz, S. G. et al., *Molecular Cell*, 4, 563-561, 1999).

### <5-2> Structure of sDR5

As a result of structural analyses of sDR5 protein in the TRAIL-sDR5 complex obtained from the Example 3, sDR5 has modular composition of N1 (residues 28-41), A1+B2 (CRD1; residues 44-84), and A1+B2 (CRD2; residues 86-125) (Fig. 2a). A1 and B2 modules are composed of 12-17 and 21-24 amino acids, respectively. They are defined by the consensus sequence Cysl-X₂-Gly-X-Tyr (or Phe)-X-X₄₋₉-Cys2 and Cys1-X₂-Cys2-X₃₋₆-X₅-Cys3-Thr-X₂₋₅-Asn-Thr-Val-Cys4 described in the SEQ. ID NO.3 and 4, respectively (Naismith, J. M. et al., *TIBS*, 23, 74-79, 1998), where 'X' in the Xₙ is one of essential amino acids and 'n' in the Xₙ is the number of intervening amino acids.

In the A1 modules, one disulfide bond between Cys1 and Cys2 exists, while Cys1 : Cys3 and Cys2 : Cys4 form two disulfide bonds in the B2 module. The N1 module has only one disulfide bond but is structurally related to the B2 module (Fig. 2b). Interestingly, in place of the N1 module in sDR5, the B2 module is located at the corresponding position in sTNFR55.

Residues 28-34 of the N1 module, which is structurally homologous to the B2 module, are involved in tight backbone interactions with the A1 module in CRD1 of sDR5, and these interactions are conserved in TNFR55. Consequently, the domain conformation and the arrangement of CRD1 relative to the Nl module in sDR5 are virtually the same as those of the corresponding CRD relative to the B2 module in sTNFR55.

The TRAIL receptors contain two repeats of extracellular CRDs, whereas other TNFR family members contain three or more CRDs (Smith, C. A. et al., *Cell*, 76:959-962, 1994). sTNFR55 is composed of four CRDs; two central CRDs corresponding to the CRDs of TRAIL receptors, the N-terminal and the C-terminal CRDs. The basic repeating structural unit of the extracellular domain is a set of smaller molecules that are the building blocks of CRDs (Bazan, J. F., *Curr. Biol.,* 3:603-606, 1993).

The Fig. 3a shows N1, CRD1 and CRD2 modules in the three-dimensional structure of TRAIL-sDR5 complex. A superposition of the structures of TRAIL-sDR5 and TNFβ-sTNFR55 discloses that the N1 module and CRD1 in sDR5 and the corresponding B2 module and CRD in TNFR55 adopt virtually the same geometry on the complex formation (Fig. 3a). The structurally similar B2 and N1 module appear to be functionally replaceable without affecting receptor-binding mode.

The domain structures of TNFR family members are very flexible, as it was first observed for STNFR55 that adapts its structure to change in solvent conditions or upon binding to TNFβ (Naismith, J. M. et al., *Structure*, 4:1251-1262, 1996). A superposition of the TRAIL-sDR5 and TNFβ-STNFR55 structures shows a clear difference in the relative orientation between CRD2 and the corresponding CRD of sTNFR55 (Fig. 3a). A torsional angle change of ∼30° occurs at the connection (Gln 85) between CRD1 and CRD2 of sDR5 compared with sTNFR55 bound to TNFβ (Fig. 3a). Apparently, the domain movement is to complement the geometric difference in the receptor-binding surfaces of the two ligands for specific interactions between pairs of residues, and probably is a common feature of the receptor family.

The Fig. 3b shows the B2 module of CRD2 of sDR5 in the three-dimensional structure of TRAIL-sDR5 complex. As illustrated in the Fig. 3b, the six sDR5 molecules in the asymmetric unit exhibit a large structural disparity in the B2 module of CRD2. While the B2 module and the successive three residues at the C-terminus in two sDR5 molecules are well ordered, those in the other four molecules are highly disordered. The positions of the two ordered B2 modules in our structure are also different from those in the previously reported structures of the complex (Fig. 3b) due to the different crystal packing. The module is a link between the putative transmembrane helix and the other part of the extracellular domain. The connection region of sTNFR55 (the C-terminal CRD) in the TNFβ-sTNFR55 complex is also highly disordered. The conformational flexibility at the connection region appears to be a general feature of the TNFR family that may help the intracellular domains to form signaling complexes with cellular partners upon ligand-binding.

### <5-3> Interface of the TRAIL-sDR5 complex

TRAIL and sDR5 form a tight 3:3 complex in the TRAIL-sDR5 complex prepared by the Example 3. Three sDR5 molecules in a curved shape bind to the three identical grooves between neighboring subunits of TRAIL (Fig. 1a). The complex formation reduces solvent-accessible surface areas by 4879 Å in TRAIL and 5058 Å in three sDR5 molecules. The binding interface between TRAIL and sDR5 can be divided into three areas similarity to that of the TNGβ-sTNFR55 complex; lower contact region, a central region, and upper contact region (Fig. 1a).

A critical feature distinguished from the two previously determined structures of TRAIL-sDR5 (Hymowitz, S. G. et al., *Cell*, 4:563-571, 1999; Mongkolsapaya, J. et al., Nature Struct. *Bio.,* 6:1048-1053, 1999), that of TNGβ-sTNFR55, and a model of TRAIL-sDR4 (Cha, S. S. et al., *Immunity,* 11:253-261, 1999) is the existence of extensive interactions in the central region, otherwise a central cavity like in the other three structures of the complexes, involves residues 131-135 of the AA" loop that penetrate into the central binding interface upon the complex formation (Fig. 1a, 4a). The segment forms several specific polar interactions with sDR5, displaying strong electron densities even in an omit map (Fig. 4b). The guanidino group of Arg 132 makes a polar interaction with Tyr 50 of sDR5. Asn 134 and Thr 135 interacts with Gln 70 and Asn 81 of sDR5, respectively (Fig. 4c). The three interacting residues of sDR5 are identical or homologous substituted in the other three TRAIL receptors.

A significant decrease in solvent-accessible surface area of residues 131-135 upon the complex formation from 526 Å to 130 Å also supports tight van der Waals' contacts with sDR5. Intriguingly, several residues after Thr-135 that should exit out of the relatively small space between the ligand and receptor exhibit barely observable electron densities. Putative courses of these residues starting from Leu-136 are surrounded by negatively charged surface (Fig. 4a). Unfavorable contact between this region and Leu-136 and the flexible nature of the first part of the loop are likely to be responsible for preventing the formation of an ordered segment.

In the two previously determined TRAIL-sDR5 structures, the course of residues 131-135 in our structure is occupied by water molecules (Hymowitz, S. G. et al., *Cell*, 4:563-571, 1999) or water molecules and the side chain of Arg-130 (Mongkolsapaya, J. et al., *Nature Struct. Bio.*, 6:1048-1053, 1999) that is located at the disallowed region in the Ramachandran plot. These residues plus the other residues of the first half of the AA" loop in the two structures are not included (Hymowitz, S. G. et al., *Cell*, 4:563-571, 1999) or poorly defined as reported (Mongkolsapaya, J. et al., *Nature Struct. Bio.*, 6:1048-1053, 1999). As a result, in the two preceding structures, a salt bridge between Arg 149 of TRAIL and Glu 94 of sDR5 is the only interaction between the AA" loop and the receptor (Hymowitz, S. G. et al., *Cell*, 4:563-571, 1999; Mongkolsapaya, J. et al., *Nature Struct. Bio.*, 6:1048-1053, 1999). The distance between two residues, which is >3.55 Å in all the three complex structures, precludes a strong ionic interaction. Accordingly, R149A mutant TRAIL shows only a slight reduction in the apoptotic activity (Hymowitz, S. G. et al., *Biochemistry*, 39:633-640, 2000). Therefore, the two previous structures preclude an assignment of any meaningful function of the novel insertion on the AA" loop. The differences in the interpretation of the electron densities at the central contact region can be due to the different crystallization conditions, although the loops in the present invention and in the other two structures do not (Hymowitz, S. G. et al., *Cell*, 4:563-571, 1999) or only weakly interact (Mongkolsapaya, J. et al., Nature *Struct. Bio.*, 6:1048-1053, 1999) with neighboring molecules in the crystals.

### Example 6: TRAIL-deletion mutant protein

It was confirmed that the residues 131-135 formed the specific polar interaction between the AA" loop of TRAIL and sDR5 receptor. So, the present inventors constructed a TRAIL mutant containing the deletion of residues 132-135 in order to examine the effect of residues 131-135 of AA" loop to the interaction of TRAIL protein and receptor.

### <6-1> Construction of TRAIL mutant protein

To compare the characteristics of mutant TRAIL with that of the wild-type TRAIL, TRAIL mutant protein containing the deletion of residues 132-135 in AA" loop was constructed by the overlapping PCR as reported (Du, Z. et al., *BioTechniques*, 18:376-378, 1995). The correct construction and expression verified by nuclotide sequencing, mass spectrometry and circular dichroism spectroscopy. The construction, expression and purification of the mutant TRAIL were done by the same method described in the Example 1.

### <6-2> Determination of receptor binding affinity

To examine the receptor binding affinity of TRAIL mutant protein constructed in the Example <6-1>, the apparent K_{Ds} values between sDR5 and wild-type and mutant TRAIL (Δ132-135) protein were measured using a BIAcore 2000 biosensor (Biosensor, Sweden).

Particularly, sDR5 (30 ug/ml in 10 mM sodium acetate, pH 4.6) was covalently bound to the carboxylated dextran matrix at a concentration of 998 response units (RU) by an amine coupling method as suggested by the manufacturer. A flow path involving two cells was employed to simultaneously measure kinetic parameters from one flow cell containing DR5-immobilized sensor chip and the other flow cell containing an underivatized chip.

For kinetic measurements, TRAIL samples, ranging from 300 to 900 nM were prepared by dilution with the HBS buffer containing 150 mM NaCl, 3 mM EDTA, 0.005% polysorbate and 10 mM HEPES (pH 7.4). Injection of each 80 ul of TRAIL solution into the flow cells (association phase) was followed by the flow of HBS buffer (dissociation phase) , both at 30 µℓ/min. Between cycles, the immobilized ligand was regenerated by injection 30 ul of 10 mM sodium phosphate buffer (pH 7.5) containing 1.0 M NaCl at 10 ul/min. All experiments were performed at 25°C. The kinetic parameters were determined by nonlinear regression analysis according to 1:1 binding model using the BIAevaluation version 2.1 software provided by the manufacturer.

When assessed by surface plasmon resonance spectroscopy, the apparent K_{D} (dissociation constant) of the deletion mutant for sDR5, which was 2.94 (± 1.61) X 10⁻⁷ M, was 18-fold higher than that (1.59 [± 0.23] X 10⁻⁸ M) of wild-type TRAIL (Fig. 5a). This result means that the residues 132-135 in AA" loop of sDR5 protein plays a key role in the binding to the sDR5 receptor.

### <6-3> Measurement of in vitro cytotoxic activity

In order to examine the effect of residues 131-135 in AA" loop of TRAIL protein to the cytotoxic acticity, the cytotoxic activities of TRAIL mutant constructed in the Example <6-1> and wild-type TRAIL protein were measured on human SK-HEP-1 hepatoma cells (ATCC HTB-52).

Briefly, a total of 50 ul of the cells in 10% FBS-DMEM media was transferred to individual wells of 96-well microplates at a density of 1 X 10⁴ cells/well. Wild-type or the mutant TRAIL diluted in the 50 ul of the same media was added to the wells at various concentration (0-1 ug/ml), and the cells were incubated for 24 hrs at 37°C. Cell viability was determined by measuring the cellular metabolic activity with 3-[4,5-dimethylthiazol-2-yl]-2,5-diphenyl tetrazolium bromide (thiazoyl blue, Sigma-Aldrich) added to the medium at a concentration of 0.5 mg/ml. Absorbance was measured at 540 nm.

Consistently, the deletion mutant showed a profound decrease in the cytotoxic activity in an *in vitro* assay using human hepatoma cells. While wild-type TRAIL reduced viability of culture cells by 50% at about 50 ng/ml concentration, the deletion mutant resulted in less than 50% cell death even at 1000 ng/ml (Fig. 5b). Therefore, the interactions at the central contact region bear direct correlation with the activity of TRAIL.

These interactions also explain the activities of previously reported TRAIL variants containing a deletion on the AA" loop, one containing the deletion of residues 137-152 and the other containing Ser-Leu-Leu sequence instead of 135-153 to mimic the short AA" loop of TNFβ. Both mutants exhibited a drastically reduced apoptotic activity (Cha, S. S. et al., *Immunity,* 11:253-261, 1999) or affinity for sDR5 (Mongkolsapaya, J. et al., *Nature Struct. Bio.*, 6:1048-1053, 1999). The weak interaction of Arg 149 alone cannot explain the defective activities. These, however, can be explained by the expected loss of the movable nature of the AA" loop when it is curtailed. Although the shortened AA" loops of the deletion mutants contain one fewer or all the residues that interact with the receptor, they cannot be translocated into the central binding region, especially because the second half of the AA" loop is held tight on the outer β-sheet of TRAIL. Together with the previous and the new deletion analyses, our structure underscores the critical role of the frame insertion in the receptor recognition played by providing the conformational flexibility of the AA" loop.

### INDUSTRIAL APPLICABILITY

The present invention demonstrates that the specific residues in the AA" loop of TRAIL protein profoundly involved in the interaction with the receptor for forming TRAIL-sDR5 complex, and the specific residues play an important role in the receptor binding affinity and the cytotoxic activity. Therefore, the three-dimensional structure of TRAIL-sDR5 complex and the specific residues in the AA" loop of the present invention can be used for the molecular strategy conferring specificity for the recognition between TNF family members and TNF receptor family members as well as for the development of TRAIL protein, which has a better stable, cytotoxic activity or an improved receptor binding affinity.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended claims.

## Claims

1. An expression vector containing sDR5 encoding gene which has the amino acid sequence described in the SEQ. ID NO. 2.

2. An *E.coli* transformant containing the expression vector of the claim 1 (Accession NO: KCCM-10230).

3. A method for producing the sDR5 protein which comprises the following steps of;
1) during the cultivation of *E.coli* transformant of the claim 2, adding IPTG as the inducer to the medium and further culturing;
2) disrupting the cells by sonication and recovering cell lysate; and
3) purifying the sDR5 protein having the amino acid sequence described in the SEQ. ID NO.2 by performing column chromatography with the cell lysate.

4. A sDR5 protein produced by the method of the claim 3.

5. A TRAIL-sDR5 complex prepared by mixing the TRAIL protein described in the SEQ. ID NO.1 of the amino acid sequence with the sDR5 protein of the claim 4.

6. A crystallizing method of TRAIL-sDR5 complex of the claim 5 which comprises the following steps of;
1) adding 1 ml of precipitant solution containing 16% polyethyleneglycol 3000, 0.05 M sodium acetate (pH 4.5), 0.55 M sodium acetate and 0.6 M sodium chloride to the well;
2) mixing the precipitant solution with the protein solution in 1:1 molar ratio on the surface of cover slip; and
3) covering the well with the cover slip of the step 2) to be crystallized the protein.

7. A crystal of TRAIL-sDR5 complex prepared by the crystallizing method of the claim 6.

8. The crystal of TRAIL-sDR5 complex according to the claim 7, which belongs to the space group P2₁ with cell dimension a = 68.63, b = 124.81, c = 128.37 Å and β = 104.49°, and the asymmetric unit contains two trimeric complexes of TRAIL and sDR5.

9. A three-dimensional structure of TRAIL-sDR5 complex of the claim 5 **characterized that** CD and EF loops of TRAIL become ordered on sDR5 binding and residues 130-145 of AA" loop display the most remarkable structural changes.

10. The three-dimensional structure of TRAIL-sDR5 complex according to the claim 9, wherein sDR5 has modular composition of N1 (residues 28-41), A1+B2 (CRD1; residues 44-84), and A1+B2 (CRD2; residues 86-125).

11. The three-dimensional structure of TRAIL-sDR5 complex according to the claim 10, wherein A1 and B2 modules are composed of the consensus sequence described in the SEQ. ID NO.3 and 4, respectively, the N1 module has only one disulfide bond, and residues 28-34 of the N1 module, which is structurally homologous to the B2 module, are involved in tight backbone interactions with the A1 module in CRD1 of sDR5.

12. The three-dimensional structure of TRAIL-sDR5 complex according to the claim 9, wherein the residues 131-135 of AA" loop, that penetrate into the central contact region of TRAIL-sDR5, are involved in the profound interaction with TRAIL receptors.

13. The three-dimensional structure of TRAIL-sDR5 complex according to the claim 9, wherein the guanidino group of Arg 132 in the residues 131-135 makes a polar interaction with Tyr 50 of sDR5, and Asn 134 and Thr 135 interacts with Gln 70 and Asn 81 of sDR5, respectively.

14. A method for developing recombinant proteins using the three-dimensional structure of the claim 9 to improve the stability or the cytotoxic activity of TRAIL protein, which comprises the following steps of;
1) changing the amino acid of AA" loop to increase the various interaction between amino acids or form the binding site of metal ion or the disulfide bonding; or
2) changing the corresponding amino acid of homotrimer interface or homodimer interface to increase the various interactions between amino acids or form the binding site of metal ion or disulfide bond, or fill the cavity.


SEQUENCE LISTINGS

<110>   DONG HWA PHARM. IND. CO., LTD

<120>   Three-dimensional structure of human derived apoptotic
        factor and receptor thereof by X-ray chrystallography and
        TRAIL deletion mutant protein

<130>   0fpo-12-14

<160>   4

<170>   KopatentIn 1.55

<210>   1
<211>   168
<212>   PRT
<213>   Homo sapiens

<400>   1
Val Arg Glu Arg Gly Pro Gln Arg Val Ala Ala His Ile Thr Gly Thr
 1               5                  10                  15

Arg Gly Arg Ser Asn Thr Leu Ser Ser Pro Asn Ser Lys Asn Glu Lys
            20                  25                  30

Ala Leu Gly Arg Lys Ile Asn Ser Trp Glu Ser Ser Arg Ser Gly His
        35                  40                  45

Ser Phe Leu Ser Asn Leu His Leu Arg Asn Gly Glu Leu Val Ile His
    50                  55                  60

Glu Lys Gly Phe Tyr Tyr Ile Tyr Ser Gln Thr Tyr Phe Arg Phe Gln
65                  70                  75                  80

Glu Glu Ile Lys Glu Asn Thr Lys Asn Asp Lys Gln Met Val Gln Tyr
                85                  90                  95

Ile Tyr Lys Tyr Thr Ser Tyr Pro Asp Pro Ile Leu Leu Met Lys Ser
            100                 105                 110

Ala Arg Asn Ser Cys Trp Ser Lys Asp Ala Glu Tyr Gly Leu Tyr Ser
        115                 120                 125

Ile Tyr Gln Gly Gly Ile Phe Glu Leu Lys Glu Asn Asp Arg Ile Phe
    130                 135                 140

Val Ser Val Thr Asn Glu His Leu Ile Asp Met Asp His Glu Ala Ser
145                 150                 155                 160

Phe Phe Gly Ala Phe Leu Val Gly
                165

<210>    2
<211>    130
<212>    PRT
<213>    Homo sapiens

<400>    2
Ala Leu Ile Thr Gln Gln Asp Leu Ala Pro Gln Gln Arg Val Ala Pro
1               5                   10                  15

Gln Gln Lys Arg Ser Ser Pro Ser Glu Gly Leu Cys Pro Pro Gly His
            20                  25                  30

His Ile Ser Glu Asp Gly Arg Asp Cys Ile Ser Cys Lys Tyr Gly Gln
        35                  40                  45

Asp Tyr Ser Thr His Trp Asn Asp Leu Leu Phe Cys Leu Arg Cys Thr
    50                  55                  60

Arg Cys Asp Ser Gly Glu Val Glu Leu Ser Pro Cys Thr Thr Thr Arg
65                  70                  75                  80

Asn Thr Val Cys Gln Cys Glu Glu Gly Thr Phe Arg Glu Glu Asp Ser
                85                  90                  95

Pro Glu Met Cys Arg Lys Cys Arg Thr Gly Cys Pro Arg Gly Met Val
            100                 105                 110

Lys Val Gly Asp Cys Thr Pro Trp Ser Asp Ile Glu Cys Val His Lys
        115                 120                 125

Glu Ser
    130


<210>    3
<211>    8
<212>    PRT
<213>    Homo sapiens

<220>
<221>    SIMILAR
<222>    (2)..(7)
<223>    2nd amino acid is two of the any amino acid, 4th amino acid
         is one of the any amino acid, 5th amino acid is Tyr or Phe,
         6th amino acid is one of the any amino acid and 7th amino
         acid is four to nine of the any amino acid

<400>    3
Cys Xaa Gly Xaa Tyr Xaa Xaa Cys
1               5


<210>    4
<211>    12
<212>    PRT
<213>    Homo sapiens

<220>

<221>   SIMILAR
<222>   (2)..(8)
<223>   2nd amino acid is two of the any amino acid, 4th amino acid
        is three to six of the any amino aicds, 5th amino acid is
        one of the amino acid and 8th amino acid is two to five of
        the any amino acid

<400>   4
Cys Xaa Cys Xaa Xaa Cys Thr Xaa Asn Thr Val Cys
1               5                   10